# EUROPÄISCHE PATENTSCHRIFT

(11) **EP 2 932 961 B1**
(45) Veröffentlichungstag und Bekanntmachung des Hinweises auf die Patenterteilung: **06.05.2020**
(21) Anmeldenummer: 15000886.0
(22) Anmeldetag: 26.03.2015
(51) Int. Cl.: A61K 9/00, A61K 31/00

(54) **VERFAHREN UND SYSTEM ZUM INTRAVAGINALEN VERABREICHEN VON PROGESTERON**
METHOD AND SYSTEM FOR INTRAVAGINAL ADMINISTRATION OF PROGESTERONE
PROCÉDÉ ET SYSTÈME D'ADMINISTRATION INTRAVAGINALE DE PROGESTÉRONE

(30) Priorität: 26.03.2014 DE 102014004388
(43) Veröffentlichungstag der Anmeldung: 21.10.2015
(73) Patentinhaber: Kayser, Antonia, 23562 Lübeck (DE)
(72) Erfinder: Kayser, Angela, 13465 Berlin (DE)
(74) Vertreter: Kayser, Christoph

(56) Entgegenhaltungen:
- EP-A1- 0 050 867
- WO-A1-2016/054002
- US-A- 5 869 081
- US-A1- 2007 077 269
- US-A1- 2008 199 511
- US-A1- 2014 005 157
- IBRAHIM M ET AL: "Progesterone supplementation for prevention of preterm labor: A randomized controlled trial", MIDDLE EAST FERTILITY SOCIETY JOURNAL, ELSEVIER, AMSTERDAM, NL, vol. 15, no. 1, 1 January 2010 (2010-01-01), pages 39-41, XP027246285, ISSN: 1110-5690 [retrieved on 2010-01-01]
- Errol R Norwitz ET AL: "Progesterone supplementation and the prevention of preterm birth", Reviews in obstetrics & gynecology, 1 January 2011 (2011-01-01), page 60, XP055598890, United States DOI: 10.3909/riog0163 Retrieved from the Internet: URL:https://www.ncbi.nlm.nih.gov/pmc/artic les/PMC3218546/pdf/RIOG004002_0060.pdf

## Beschreibung

Die vorliegende Erfindung betrifft ein vaginal in einen Körper einführbares Trägerlement zur Verwendung in der Frühgeburtsprophylaxe.

Aus US 5,869,081 A ist ein Verfahren bekannt, das bei einer künstlichen Befruchtung, einschließlich einer in-vitro-Fertilisation oder einem Embryotransfer, angewendet werden soll. Eine künstliche Befruchtung wird gemäß D1 auch bei Frauen durchgeführt, die funktionell keine Geschlechtsdrüsen haben. Diese Frauen können nach einer künstlichen Befruchtung anfangs kein körpereigenes Progesteron produzieren. Erst ab der achten bis zehnten Woche übernimmt die Plazenta diese Funktion. In dieser Anfangsphase wird solchen Frauen ein Vaginalring zur intravaginalen Verabreichung von Progesteron eingesetzt.

Aus EP 0 050 867 A1 ist ein Vaginalring bekannt, mit dem z.B. das Verfahren aus US 5,869,081 A durchgeführt werden kann.

Aus US 2007/0077269 A1 ist ein Verfahren bekannt, bei dem im Rahmen der Empfängnisverhütung ein Vaginalring während eines Menstruationszyklus getragen werden soll, über den u.a. eine Progesteron-Verbindung verabreicht wird, um den Hormonhaushalt der Trägerin zu regulieren. Das Verfahren dient als Ersatz für die Pille.

Aus US 2008/0199511 A1 ist ein Verfahren bekannt, bei dem durch Verabreichung von Progesteron-Rezeptoren über Vaginalring eine langzeitige Schwangerschaftsverhütung erreicht wird.

Aus Errol R. Norwitz ET AL: "Progesterone supplementation and the prevention of preterm birth",Reviews in obstetrics & gynecology, 1. Januar 2011 (2011 -01 -01), S. 60-72 ist ein Verfahren bekannt, bei dem schwangeren Frauen zur Prävention einer Frühgeburt der Wirkstoff Progesteron in verschiedenen pharmazeutischen Formen verabreicht wird.

Aus dem Stand der Technik ist bekannt, bei schwangeren Frauen zur Sekundärprävention einer Frühgeburt den Wirkstoff Progesteron mit sich im Körper auflösenden Kapseln vaginal einzuführen. Solche Kapseln werden zum Beispiel unter der Marke Utrogest® von Dr. Kade/Besins Pharma GmbH vertrieben.

Progesteron ist ein weibliches Sexualhormon aus der Gruppe der Gestagene, das einerseits in der Frühschwangerschaft zur Abortverhinderung, andererseits in der späteren Schwangerschaft zur Frühgeburtsprophylaxe eingesetzt wird. Der größte Fortschritt in der Frühgeburtsprävention des letzten Jahrzehnts ist die Einführung der Progesteronsubstitution. Das Risiko für eine Frühgeburt kann um mehr als 30% sowohl bei Frauen mit belasteter Anamnese als auch bei aktueller Zervixverkürzung signifikant gesenkt werden. Evidenzbasiert sollte jeder betroffenen Schwangeren eine Progesteronsubstitution bis zur vollendeten 34.

Schwangerschaftswoche empfohlen werden.

Die vaginale Anwendung hat den Vorteil, dass der Wirkstoff Progesteron besser wirkt und auch besser verträglich ist. Für den Fall, dass eine Substitution medizinisch erforderlich ist, muss die vorgenannte Kapsel täglich (mehrfach) verabreicht werden. Damit die Patientin nicht ebenso häufig ihren Arzt aufsuchen muss, erhält sie eine kleine Menge Kapseln mit nach Hause, um sich diese täglich selbst vaginal einzuführen. Bei der selbsttätigen Einführung der Kapsel in ihre Vagina, muss die Patientin darauf achten, dass der Vorgang stets steril abläuft. Bei einer nicht sterilen Vorgehensweise können Keime die günstige Wirkung des Progesterons auf den Verlauf der Schwangerschaft zunichte machen und erst recht eine Frühgeburt auslösen. Keime erhöhen das Risiko einer Fehlgeburt erheblich, auch wenn Progesteron intravaginal verabreicht wird.

Die Aufgabe der vorliegenden Erfindung ist daher, eine für die Patientin zur Frühgeburtsprophylaxe eine komfortable Möglichkeit zu schaffen, über längere Zeiträume mit dem Wirkstoff Progesteron steril versorgt zu werden.

Die Aufgabe wird erfindungsgemäß durch die Verwendung des Trägerelements gemäß Anspruch 1 gelöst.

Wenn der Wirkstoff Progesteron auf einem nicht resorbierbaren Trägerelement bereitgestellt wird und dieses nicht resorbierbare Trägerelement lösbar über eine vorbestimmte Zeitdauer in der Vagina einer Patientin eingesetzt werden kann, ist es möglich, dass das Trägerelement den Wirkstoff dosiert über eine vorbestimmte Zeitdauer abgibt. Es ist ein großer Vorteil, dass die Patientin nicht mehr täglich ein resorbierbares Trägerelement mit dem Wirkstoff Progesteron selbsttätig einführen muss. Das Risiko durch eine nicht sorgfältige Beachtung der sterilen Vorgehensweise entfällt damit vollends. Die täglichen Arztbesuche sind auch nicht mehr nötig, da eine längere Zeitdauer vorbestimmt werden kann, über die der Wirkstoff Progesteron abgegeben wird. Das nicht resorbierbare Trägerelement muss also erst nach mehreren Tagen oder gar Wochen ausgetauscht werden.

Ein weiterer Vorteil der vorliegenden Erfindung ist, dass das Trägerelement als Vaginalring bereitgestellt wird. Solche Vaginalringe sind an sich bekannt und mit anderen Wirkstoffen dotiert. Ein solcher bekannter Vaginalring ist für die Patientin bequem zu tragen und von medizinischem Personal leicht zu entfernen.

In einer bevorzugten Ausführungsform ist das nicht resorbierbare Trägerelement ein an sich bekannter Vaginalring oder Pessar. Ein im Handel erhältlicher Vaginalring ist zum Beispiel der NuvaRing® von der MSD Sharp & Dohme GmbH.

In anderen Ausführungsformen können auch andere nicht resorbierbare Trägerelemente für das erfindungsgemäße Verfahren und das erfindungsgemäße System zum Einsatz kommen. Das erfindungsgemäße, nicht resorbierbare Trägerelement wird zur Durchführung des erfindungsgemäßen Verfahrens mit dem Wirkstoff Progesteron dotiert. Das mit dem Wirkstoff Progesteron dotierte, nicht resorbierbare Trägerelement wird dann bei der Patientin von medizinischem Personal vaginal steril eingeführt und in der Patientin so positioniert, dass dieses dort intravaginal für eine vorbestimmte Zeitdauer verbleiben kann. Als eine vorbestimmte Zeitdauer werden wenigstens mehrere Tage ausgewählt. Der Verabreichung des Wirkstoffs Progesteron zur Frühgeburtsprävention über ein nicht resorbierbares Trägerelement soll tägliche Verabreichungen einer Tagesdosis durch eine Verabreichung jeweils mehrtägiger Tagesdosen ersetzen, wobei die Höhe der Dosis und der zeitliche Abstand der Verabreichung im Einzelfall vom Behandler festzulegen ist. Wenigstens mehrere Tage bedeuten hier also wenigstens 2 Tage und bis zu so vielen Tagen, bis die Patientin ohne wieder routinemäßig zu einer Kontrolle gehen muss.

Damit der Verbleib des nicht resorbierbaren Trägerelements an der Innenwand der Vagina gewährleistet ist, sitzt das nicht resorbierbare Trägerelement vorzugsweise form- und kraftschlüssig an der Innenwand der Vagina. Wenn das Trägerelement in der bevorzugten Ausführungsform ein Vaginalring ist, dann schmiegt sich dieser an die Innenwand der Vagina an.

Das nicht resorbierbare Trägerelement, zum Beispiel der Vaginalring, ist so ausgebildet, dass dieser den Wirkstoff Progesteron dosiert über die vorbestimmte Zeitdauer in die Vaginalschleimhaut abgibt. Die Ausbildung des nicht resorbierbaren Trägerelements und insbesondere des Vaginalrings für eine dosierte Abgabe von Wirkstoffen über längere Zeiträume ist allgemein bekannt und nicht Gegenstand der vorliegenden Erfindung.

## Patentansprüche

1. Vaginal in einen Körper einführbares Trägerelement zur Verwendung für die Frühgeburtsprophylaxe in der Schwangerschaft,
wobei Progesteron in mehreren Tagesdosen auf einem nicht resorbierbaren Trägerelement bereitgestellt und vaginal eingeführt wird,
wobei das nicht resorbierbare Trägerelement intravaginal für eine vorbestimmte Zeitdauer eingesetzt ist und Progesteron freisetzt, und
wobei als die vorbestimmte Zeitdauer mehrere Tage ausgewählt werden.

2. Progesteron haltiges Trägerelement nach Anspruch 1 zur Verwendung für die Frühgeburtsprophylaxe in der Schwangerschaft,
**dadurch gekennzeichnet,**
**dass** das Trägerelement form- und kraftschlüssig an der Innenwand der Vagina angelegt wird.

3. Progesteron haltiges Trägerelement nach Anspruch 1 oder 2 zur Verwendung für die Frühgeburtsprophylaxe in der Schwangerschaft,
**dadurch gekennzeichnet,**
**dass** als Trägerelement als Vaginalring bereitgestellt wird.

4. Progesteron haltiges Trägerelement nach einem der vorstehenden Ansprüche zur Verwendung für die Frühgeburtsprophylaxe in der Schwangerschaft,
**dadurch gekennzeichnet,**
**dass** das Trägerelement über mehrere Wochen, vorzugsweise bis zur vollendeten 34. Schwangerschaftswoche in der Vagina verbleibt.

5. Progesteron haltiges Trägerelement nach einem der vorstehenden Ansprüche zur Verwendung für die Frühgeburtspröphylaxe in der Schwangerschaft
**dadurch gekennzeichnet,**
**dass** das Trägerelement den Wirkstoff Progesteron dosiert über die vorbestimmte Zeitdauer abgibt.

## Claims

1. A carrier element insertable vaginally into a body for use in preventing premature birth in pregnancy, wherein progesterone is provided in a number of daily doses on a non-resorbable carrier element and is introduced vaginally,
wherein the non-resorbable carrier element is inserted intravaginally for a predetermined period of time and releases progesterone, and
wherein a number of days are selected as the predetermined period of time.

2. The progesterone-containing carrier element according to claim 1 for use in preventing premature birth in pregnancy,
**characterised in that**
the carrier element is placed against the inner wall of the vagina form-fittingly and frictionally.

3. The progesterone-containing carrier element according to claim 1 or 2 for use in preventing premature birth in pregnancy,
**characterised in that**
a vaginal ring is provided as carrier element.

4. The progesterone-containing carrier element according to any one of the preceding claims for use in preventing premature birth in pregnancy,
**characterised in that**
the carrier element remains in the vagina for a number of weeks, preferably until the end of the 34^{th} week of pregnancy.

5. The progesterone-containing carrier element according to any one of the preceding claims for use in preventing premature birth in pregnancy,
**characterised in that**
the carrier element releases the active substance progesterone in dosed fashion over the predetermined period of time.

## Revendications

1. Élément de support susceptible d'être introduit par voie vaginale dans un corps, pour une utilisation pour la prophylaxie des accouchements prématurés pendant la grossesse,
de la progestérone étant mise à disposition en plusieurs doses journalières sur un élément de support non résorbable et introduite par voie vaginale,
l'élément de support non résorbable étant inséré pour une période prédéfinie par voie intravaginale et libérant de la progestérone et plusieurs jours étant choisis comme étant la période prédéfinie.

2. Élément de support contenant de la progestérone selon la revendication 1, pour une utilisation pour la prophylaxie des accouchements prématurés pendant la grossesse,
**caractérisé en ce que**
l'élément de support est posé par complémentarité de forme et de force sur la paroi interne du vagin.

3. Élément de support contenant de la progestérone selon la revendication 1 ou 2 pour une utilisation pour la prophylaxie des accouchements prématurés pendant la grossesse,
**caractérisé en ce que**
l'élément de support est mis à disposition sous la forme d'un anneau vaginal.

4. Élément de support contenant de la progestérone selon l'une quelconque des revendications précédentes pour une utilisation pour la prophylaxie des accouchements prématurés pendant la grossesse,
**caractérisé en ce que**
l'élément de support reste pendant plusieurs semaines, de préférence jusqu'à la fin de la 34^{ème} semaine de grossesse dans le vagin.

5. Élément de support contenant de la progestérone selon l'une quelconque des revendications précédentes pour une utilisation pour la prophylaxie des accouchements prématurés pendant la grossesse
**caractérisé en ce que**
l'élément de support délivre le principe actif progestérone de manière dosée sur la période prédéfinie.
